# EUROPEAN PATENT SPECIFICATION

(11) **EP 2 780 023 B1**
(45) Date of publication and mention of the grant of the patent: **03.01.2018**
(21) Application number: 12797955.7
(22) Date of filing: 16.11.2012
(51) Int. Cl.: A61K 35/744, A61K 35/747, A61K 36/8998, A61P 1/00, A61P 1/04, A61P 1/10, A61P 1/12

(54) **IBS TREATMENT WITH PROBIOTICS**
IBS-BEHANDLUNG MIT PROBIOTIKA
TRAITEMENT DU SYNDROME DU CÔLON IRRITABLE (IBS) PAR DES PROBIOTIQUES

(30) Priority: 16.11.2011 GB 201119774; 09.12.2011 GB 201121207
(43) Date of publication of application: 24.09.2014
(60) Divisional application: 17198980.9
(73) Proprietor: Multigerm UK Enterprises Ltd., Surrey GU10 1PX (GB)
(72) Inventor: SMITH, Barry, Edward, Farnham Surrey GU10 1PX (GB)
(74) Representative: Boult Wade Tennant
(86) International application number: PCT/GB2012/000841
(87) International publication number: WO 2013/072654

(56) References cited:
- WO-A1-2006/035218
- WO-A1-2007/036230
- WO-A1-2011/078781
- CHARALAMPOPOULOS D ET AL: "Evaluation of the effect of malt, wheat and barley extracts on the viability of potentially probiotic lactic acid bacteria under acidic conditions", INTERNATIONAL JOURNAL OF FOOD MICROBIOLOGY, ELSEVIER SCIENCE PUBLISHERS, AMSTERDAM, NL, vol. 82, no. 2, 25 April 2003 (2003-04-25) , pages 133-141, XP002353394, ISSN: 0168-1605, DOI: 10.1016/S0168-1605(02)00248-9
- TOPPING ET AL: "Cereal complex carbohydrates and their contribution to human health", JOURNAL OF CEREAL SCIENCE, ACADEMIC PRESS LTD, XX, vol. 46, no. 3, 26 October 2007 (2007-10-26), pages 220-229, XP022316971, ISSN: 0733-5210, DOI: 10.1016/J.JCS.2007.06.004
- QUIGLEY EAMONN M M: "Irritable bowel syndrome and inflammatory bowel disease: interrelated diseases?", CHINESE JOURNAL OF DIGESTIVE DISEASES 2005, vol. 6, no. 3, 2005, pages 122-132, XP002691032, ISSN: 1443-9611

## Description

### Field of the invention

The invention relates to probiotic preparations containing viable, metabolically active probiotic bacteria for use in the treatment of Irritable Bowel Syndrome.

### Background to the invention

Irritable Bowel Syndrome is the most common of all gastrointestinal diseases and is prevalent in around 10-15% of the UK population at any given time. It accounts for about 40% of all referrals to hospital based gastroenterologists in the UK (and elsewhere) and worryingly, once the diagnosis is made, over 90% of patients are still symptomatic five years later.

Affecting twice as many women as men, IBS usually begins in the late teens to twenties and fluctuates in intensity. Due to the nature of the symptoms it can be embarrassing to discuss and as a result, the percentage of people suffering from IBS at any given time is likely to be higher than reported. Key symptoms include, stomach cramps and pain, diarrhoea, constipation and bloating, with many patients being defined as having either 'diarrhoea' or 'constipation' predominant IBS.

IBS causes a great deal of discomfort and distress for many people and whilst it is not life-threatening, it can take a serious toll on the quality of an individual's life. People are increasingly taking longer periods of time off work due to IBS and with so many of the population affected the scale of problem on the economy and the burden on healthcare provision is considerable.

Researchers have yet to discover any specific cause for IBS. Theories include reactions to food, stress, abnormal motility or visceral hyper-sensitivity, the absorption of too much fluid in the digestive tract, bacterial overgrowth and the overuse of antibiotics, to name a few. As a result, GPs and gastroenterologists find it difficult to advise a course of action. Guidelines recommend treating the symptoms rather than trying to find the cause. The available treatment options using existing psychotropic agents, serotonin receptor agonists and antagonists, are limited to the most severe cases and their use is not without side effects. Some drugs prescribed for IBS are known to cause dependency, sickness or extreme drowsiness and in a few cases, agents have been taken off the market by pharmaceutical manufacturers due to serious adverse affects. For patients who do not fall into the 'severe' category and where no dietary intolerance is shown following tests, GPs can only offer general guidance on diet, lifestyle and possible over-the-counter treatments.

This leaves the majority of people suffering from IBS with little option but to try and find a solution themselves. For a lot of people, it is common to attempt to control their symptoms with either/or a combination of better diet, stress management, and over-the-counter medicines or alternative remedies. However, many only find limited relief. It is not unusual for a person with IBS symptoms to buy and consume numerous products for their condition and to spend significant amounts of money in pursuit of relief. These include antispasmodic drugs although many are associated with substantial anticholinergic side effects. People suffering with constipation dominant IBS usually start off with laxatives and those who are diarrhoea predominant try obstipants. However, almost all of these treatments are of unproven efficacy in the treatment of IBS, or are associated with significant side effects. Many patients, having tried these treatments repeatedly over a period of time, remain dissatisfied and a large number seek alternative approaches such as a change in diet, or complementary therapies.

Some patients try a new diet approach such as a high or low fibre diet, or elimination of certain foods, for example, wheat. A role for dietary triggers in IBS has never been convincingly demonstrated, although many patients feel a benefit of the dietary change. Study results over the last decade, particularly since the introduction of the Gold Standard Rome Criteria for assessing IBS, show that making these changes has limited impact in patients with IBS. Many reported little change in bowel frequency but increased gas and distension when following high fibre diets or using fibre supplementation. Other exclusion diets include gluten and lactose or milk proteins and whilst there may be improvement for some, for the majority, IBS symptoms remain.

As a result, most people have to try and live with their symptoms, although in many studies, people have mentioned that IBS has led them to curtail the way they 'normally' live, or caused them to miss out on social or family activities, stopped them from going to work, with some people also suffering from depression.

In the last decade, people's knowledge of gut health, its importance in relation to the immune system and other vital body functions, has undergone a huge change. The proliferation of articles and studies available on the internet, health campaigns directed by the NHS and charitable organisations, as well as features in consumer publications, have all helped to increase people's understanding and awareness of the role of gut flora. In a study published by a European led consortium MetaHIT in 2010, scientists reported that we all share a core group (several hundred) of different microbial genes which are responsible for at least 600 biochemical functions. The study showed that people with diseases had an imbalance of gut flora with fewer microbial genes and this could also be related to those found with GI conditions.

Probiotics come in several formats and the principle challenge is delivering the bacteria effectively and documenting a health benefit clinically in appropriately designed trials. Calculating a specific daily amount or viable colony-forming unit (CFU) at a level proven to confer a health benefit is difficult. However, it is even harder to produce a product that can cope with changes in ambient temperatures, or the pH levels of the host environment and still hold the same number of viable count at - the point where they are needed - and not at the time of manufacture. For dairy and freeze-dried probiotic formats, this is particularly challenging as yoghurts trigger digestion in the stomach, and powders and capsules need a few hours to rehydrate before they can be activated.

Until today, format may be one of the reasons why probiotic companies have struggled to prove efficacy for IBS supported by strong evidence.

Microbiological organisms are frequently used as food supplements. One example are probiotic bacteria which are known to have beneficial effects on the intestinal microflora increasing the resistance to infectious disease such as diarrhoea. Probiotics have also been shown to be involved in the modification of blood chemistry and in immunomodulation (see references listed under Potential Health Benefits in the reference section).

Probiotic bacteria can be found in dairy products such as yoghurt and species known to have health benefits include those from the genera *Enterococcus* and *Lactobacillus*. However, probiotic dairy products have a short shelf life.

Current methods for storing bacterial cultures typically use lyophilization, also called freeze-drying. In this process, water is removed from the organism by sublimation and the organism and can be revived after the addition of water. However, freeze-dried bacteria are not metabolically active and it is well known that freeze-dried products typically lose much of their resilience after a few weeks of storage at room temperature (Fonseca et al and Murga et al).

Furthermore, many commercial probiotics do not seem to contain all of the species mentioned on the labels, and where bacteria are present the numbers of viable bacteria are often very low (J. Hamilton-Miller).

### Summar of the invention

A double-blind, placebo-controlled clinical study was carried out in a cohort of patients with particularly severe irritable bowel syndrome using a non-diary liquid-based probiotic preparation which is characterised by the presence of a high count of viable, metabolically active probiotic bacteria. This clinical study clearly demonstrated a statistically significant improvement in IBS symptom severity index, following a 3 month period of treatment with the probiotic preparation.

Therefore, in accordance with a first aspect of the invention there is provided a probiotic preparation comprising viable, metabolically active probiotic bacteria in a non-dairy liquid substrate, for use in the treatment of Irritable Bowel Syndrome.

In one embodiment the probiotic preparation is capable of delivering viable, metabolically active probiotic bacteria to the intestinal tract of a human subject without triggering digestion.

In one embodiment the probiotic bacteria in the probiotic preparation are stable when maintained in culture at pH 3.0 for a period of at least 6 hours.

In accordance with a second aspect of the invention there is provided a probiotic preparation comprising viable, metabolically active probiotic bacteria in a non-dairy liquid substrate comprising a mixture of complex carbohydrates and simple sugars, wherein the ratio of total carbohydrate content to reducing sugar content of the probiotic preparation is in the range of from 8:1 to 2:1, for use in the treatment of Irritable Bowel Syndrome.

The probiotic preparation used in the treatment of IBS as described herein may be prepared by growing one or more strains of probiotic lactic acid bacteria in a growth substrate derived from malted cereal grains. More specifically, the probiotic preparation may be prepared by growing one or more strains of probiotic lactic acid bacteria in an extract of germinated barley.

Therefore, in a further aspect the invention provides a probiotic preparation comprising viable, metabolically active probiotic bacteria in a non-dairy liquid substrate, for use in the treatment of Irritable Bowel Syndrome, wherein the probiotic preparation is prepared by growing one or more strains of probiotic bacteria in an extract of germinated barley.

The invention further provides use of a probiotic preparation comprising viable, metabolically active probiotic bacteria in a non-dairy liquid substrate in the manufacture of a medicament for treating or preventing Irritable Bowel Syndrome.

### Brief description of the drawings

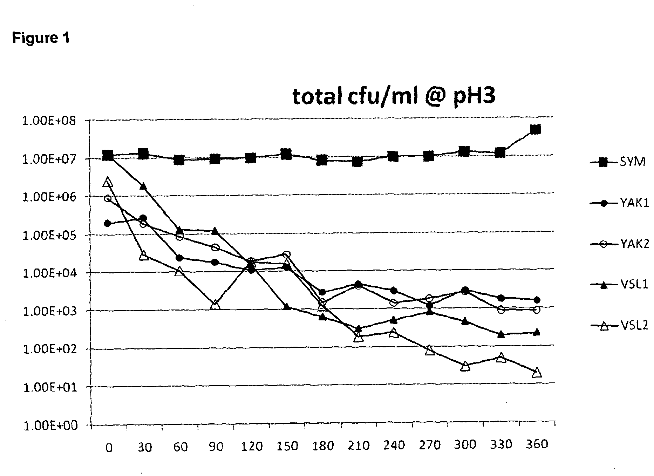

Figure 1 shows the total colony forming units per millilitre (cfu/ml) over time (in minutes) during a comparison between a commercially available multi-strain freeze-dried probiotic product (MS-FD1 & MS-FD2 in the figure), a popular commercially available single strain milk-based probiotic product (SS-MB1 & SS-MB2 in the figure) and the probiotic preparation of example 3 (Symprove™ containing multiple strains of bacteria)(SYM in the figure). The comparison was carried out at pH3.

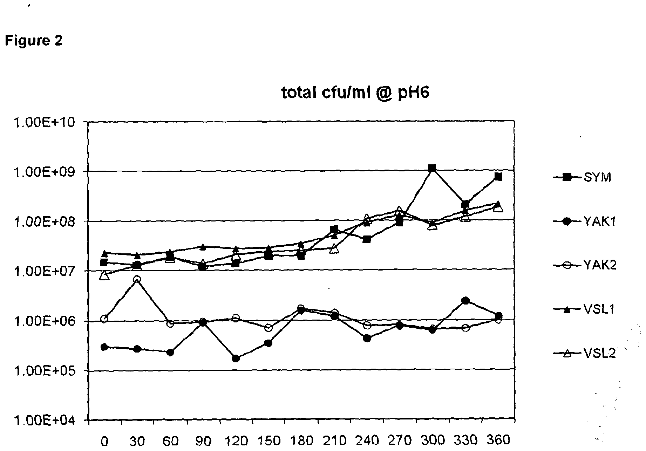

Figure 2 shows the total colony forming units per millilitre (cfu/ml) over time (in minutes) during a comparison between the multi-strain freeze-dried probiotic product (MS-FD1 & MS-FD2 in the figure), a the single strain milk based probiotic product (SS-MB1 & SS-MB2 in the figure) and the probiotic preparation of example 3 (Symprove™ containing multiple strains of bacteria)(SYM in the figure). The comparison was carried out at pH6.

### Detailed description of the invention

The invention provides effective treatment of irritable bowel syndrome (IBS) using a probiotic preparation which has been demonstrated, in a placebo-controlled, double-blind clinical study, to provide a statistically unprecedented reduction in IBS symptom severity, measured on the IBS symptom severity index.

The probiotic preparation used in the effective treatment of IBS is a liquid-based product (and typically a water-based product) which is non-dairy and is not freeze-dried. The probiotic preparation contains viable, metabolically active probiotic bacteria in a liquid substrate which is capable of delivering viable, metabolically active probiotic bacteria to the intestinal tract, where the probiotic bacteria rapidly begin to establish and multiply. The probiotic bacteria in the preparation are therefore "alive" and ready to function immediately after the preparation is swallowed.

Prior art probiotic products are predominately available in dairy or freeze-dried formats such as powders or capsules. In order for probiotics to work effectively and to their optimum, they need to be directed to specific sites within the intestinal tract without triggering digestion as well as avoiding extremes. If digestion is triggered, the stomach acids can weaken or destroy probiotic bacteria. For some probiotics this is a challenge as they are contained in yoghurt type drinks which the stomach initially 'sees' as food. On the other hand, freeze-dried probiotics have been taken down to a temperature of around minus 80°C and the fimbria can be broken off during the freezing process. Freeze-dried probiotics have to rehydrate before they are able to function adequately. This can take several hours. Finding an effective "transport system" for probiotic bacteria is a key objective for manufacturers producing probiotic supplements or functional food.

The probiotic preparation described herein and shown to be clinically effective in the treatment of severe IBS does not suffer from these drawbacks, as the probiotic bacteria are carried in a liquid-based substrate, typically a water-based substrate, which shields and transports the probiotic bacteria safely through the stomach (without triggering digestion) to the intestines where they rapidly begin to establish and multiply. Without wishing to be bound by any particular theory, it is believed that the viable nature of the probiotic bacteria in the preparation described herein and shown to be clinically effective in the treatment of IBS allows them to establish rapidly in the patient's gastrointestinal tract, perhaps within 15 to 20 minutes of ingestion. It is believed that such rapid establishment contributes to the unprecedented efficacy observed in the clinical treatment of IBS, resulting in a statistically significant reduction in IBS symptom severity.

### Definitions

"Probiotic" - as used herein the term "probiotic" is to be interpreted according to the FAO/WHO joint report and guidelines for use of probiotics, in which probiotics are defined as "live microorganisms which when administered in adequate amounts confer a health benefit to the host". The term "probiotic bacteria" refers to any bacterial strain which fulfils this definition of a "probiotic".

"Lactic acid bacteria (LAB)" - as used herein the term "lactic acid bacteria (LAB)" refers to a group of Gram positive, catalase negative, non-motile anaerobic bacteria that ferment carbohydrates to lactic acid. This group includes the genera *Lactobacillus*, *Lactococcus, Pediococcus, Bifidobacterium, and Enterococcus.*

"Probiotic lactic acid bacteria" - as used herein the term "probiotic lactic acid bacteria" refers to lactic acid bacteria which also satisfy the definition of a "probiotic" as used herein. Exemplary probiotic lactic acid bacteria include, but are not limited to, those in the genera *Lactobacillus* and *Enterococcus.*

"Irritable Bowel Syndrome" - Irritable Bowel Syndrome is a disorder with chronic or recurrent colonic symptoms. The disorder is manifested in a variety of symptoms, which may vary from patient to patient. Key symptoms include, stomach cramps and pain, diarrhoea, constipation and bloating, with many patients being defined as having either 'diarrhoea' or 'constipation' predominant IBS. The term "Irritable Bowel Syndrome" as used herein is intended to encompass all forms of this condition. The IBS Rome Criteria may be used for diagnostic purposes.

### The IBS Rome Criteria

The Rome Foundation is an independent, not for profit organisation that provides support for activities designed to create scientific data and educational information to assist in the diagnosis and treatment of functional gastrointestinal disorders. The IBS Rome II and III criteria were defined and recently updated by leading international gastroenterologists to assist clinicians in the improved diagnosis and management of IBS. The updated Rome III criteria allow for the presence of IBS symptoms over a shortened timeframe.

The Rome criteria define IBS by the presence of abdominal pain associated with altered bowel habits, in the absence of an identifiable structural or biochemical disorder. Key symptoms include, stomach cramps and pain, diarrhoea, constipation and bloating, with many patients being defined as having either 'diarrhoea' or 'constipation-predominant IBS'.

These criteria are valuable for the accurate management of clinical trials.

### Irritable Bowel Syndrome Rome II Criteria

Stomach pain or discomfort for 12 consecutive weeks in the preceding 12 months where two or three features present:
- relief by a bowel movement
- onset associated with a change in stool frequency
- onset associated with a change in the appearance or form of stool

The following cumulatively support the diagnosis of IBS:
- abnormal stool frequency
- abnormal stool form
- abnormal stool passage
- passage of mucous

### Irritable Bowel Syndrome Rome III Criteria

### Diagnostic criterion*

Recurrent abdominal pain or discomfort** at least 3 days/month in the last 3 months associated with two or more of the following:
- Improvement with defecation
- Onset associated with a change in frequency of stool
- Onset associated with a change in form (appearance) of stool

* Criterion fulfilled for the last 3 months with symptom onset at least 6 months prior to diagnosis
** "Discomfort" means an uncomfortable sensation not described as pain. In pathophysiology research and clinical trials, a pain/discomfort frequency of at least 2 days a week during screening evaluation is recommended for subject eligibility.

"Treatment" of IBS - as used herein in the specific context of IBS, the term "treatment" refers to an improvement in one or more symptoms associated with IBS. This may be measured in terms of an improvement in quality of life symptom score or an improvement on the IBS symptom severity index (i.e. reduction of symptom severity), or both.

"Complex carbohydrates" - as used herein the term "complex carbohydrates" includes both oligosaccharides and polysaccharides. "Oligosaccharides" are saccharide polymers containing 3 to 10 saccharide units; whereas the term "polysaccharides" includes longer polymeric structures, such as those formed from repeating saccharide (or disaccharide) units.

"Simple sugars" - as used herein the term "simple sugars" refers to both monosaccharides and disaccharides, unless otherwise stated.

"Reducing sugars" - as used herein the term "reducing sugars" refers to sugars which either have an aldehyde group or are capable of forming an aldehyde group in solution through isomerisation. The presence of reducing sugars may be determined by means of the Nelson-Somogyi method using glucose as the reference standard (Somogyi, M. (1052) Journal of Biological Chemistry., Vol. 195., p.19; reproduced in many standard textbooks of carbohydrate chemistry). Although certain complex carbohydrates (e.g. starches) may contain reducing ends, and therefore fulfil the definition of "reducing sugars", a determination of the content of "reducing sugars" in a given sample (e.g. a sample of probiotic preparation as described herein) using the Nelson-Somogyi method may be taken as an approximation of the amount of simple sugars in the sample, since the simple sugars contain a greater proportion of reducing ends per unit mass than complex carbohydrates.

"Total carbohydrate content" - as used herein the terms "total carbohydrate" or "total carbohydrate content" refer to the total amount of complex carbohydrate and simple sugars present in a given product (e.g. a probiotic preparation as described herein). Total carbohydrate content may be measured using the phenol-sulphuric acid assay, using glucose as a reference standard (Dubois, M., Gilles, K. A., Hamilton, J. K., Rebers, P. A. and Smith, F. (1956) Analytical Chemistry, vol. 28., p. 350).

Where reference is made herein to the ratio of total carbohydrate content to reducing sugar content of a liquid-based product (e.g. in a probiotic preparation) then this is to be determined by calculating the ratio of total carbohydrate content of the product, as measured by the phenol-sulphuric acid method described herein (result expressed in mg/ml), to reducing sugar content of the product, as measured by the Nelson-Somogyi method described herein (result expressed in mg/ml).

"Non-dairy" - as used herein the term "non-dairy" refers to products which do not contain and are not based upon milk from a mammal. Accordingly, non-dairy products do not contain and are not based upon milk, butter, cheese (including vegetarian cheese), yoghurt, cream, milk powder, whey, lactose, lactoproteins (including caseins and caseinates), anhydrous milk fat or kephir.

### Probiotic bacterial strains

The probiotic bacteria included in the probiotic preparation comprise at least the species selected from the group consisting of: *Enterococcus faecium*, *Lactobacillus plantarum, Lactobacillus casei, Lactobacillus acidophilus* and *Lactobacillus rhamnosus.*

Reference to *Lactobacillus casei* is intended to include any bacterial strain originally classified as *Lactobacillus casei* but now re-classified as *Lactobacillus rhamnosus.* Accordingly, reference to *Lactobacillus rhamnosus* may include bacterial strains which have previously been classified as *Lactobacillus casei*. A combination of *Enterococcus faecium, Lactobacillus plantarum, Lactobacillus acidophilus,* and either *Lactobacillus casei* or *Lactobacillus rhamnosus* or both *Lactobacillus casei* and *Lactobacillus rhamnosus* is used.

The product Symprove™ (containing *Enterococcus faecium, Lactobacillus plantarum, Lactobacillus casei* and *Lactobacillus acidophilus*) was shown to be particularly effective in reducing IBS symptom severity, and also improving quality of life scores for IBS patients, in the clinical study described herein. The strain of *Lactobacillus* casei in Symprove™ was originally characterised as *Lactobacillus casei* but has now been re-classified as the closely related *Lactobacillus rhamnosus.*

A key factor in achieving therapeutic efficacy in the treatment of IBS, as measured as a reduction in IBS symptom severity on the IBS symptom severity index, is the ability to deliver a high count of viable, metabolically active probiotic bacteria to the gastrointestinal tract of the patient undergoing treatment. In order to achieve this, in exemplary embodiments, the total population of metabolically active bacteria in the probiotic preparation may be in the range of from 1.0 x 10⁶ to 1.0 x 10⁹ viable cells per millilitre, preferably in the range of from 1.0 x 10⁷ to 1.0 x 10⁹ viable cells per millilitre. Each individual strain of metabolically active bacteria present in the probiotic preparation may be present in the range of from 1.0 x 10⁵ to 1.0 x 10⁹ viable cells per millilitre, more preferably in the range of from 1.0 x 10⁷ to 1.0 x 10⁹ viable cells per millilitre.

In the case of *Enterococcus faecium, Lactobacillus plantarum*, and either *Lactobacillus casei* or *Lactobacillus rhamnosus* or both *Lactobacillus casei* and *Lactobacillus rhamnosus,* it is preferred that at least one and preferably each of these strains is present in the range of from 1.0 x 10⁶ to 1.0 x 10⁹ viable cells per millilitre, preferably in the range of from 1.0 x 10⁷ to 1.0 x 10⁹ viable cells per millilitre

The population of L. *acidophilus,* may be lower than 1.0 x 10⁵ viable cells per millilitre.

In an exemplary embodiment the preparation may comprise a combination of *Enterococcus faecium, Lactobacillus plantarum*, and either *Lactobacillus casei* or *Lactobacillus rhamnosus* or both *Lactobacillus casei* and *Lactobacillus rhamnosus,* wherein the bacterial count for each of these bacterial strain is in the range of from 1.0 x 10⁵ to 1.0 x 10⁹ viable cells per millilitre, more preferably in the range of from 1.0 x 10⁷ to 1.0 x 10⁹ viable cells per millilitre, and further contains *Lactobacillus acidophilus.* The population of *L. acidophilus* may be lower than 1.0 x 10⁵ viable cells per millilitre.

Although the total bacterial count in the presently described preparation may appear to be lower than that of typical freeze-dried probiotic preparations, it is important to note that the bacteria are viable. As noted elsewhere herein, it is believed that the viable nature of the bacteria in the probiotic preparation described herein and shown to be effective in treating IBS allows them to establish more rapidly in a patient's gastrointestinal tract, perhaps within 15 to 20 minutes of ingestion. It is believed that such rapid establishment contributes to the beneficial effects of the preparation in the treatment of IBS.

Another key advantage of the probiotic preparation described herein, and shown to be useful in the treatment of IBS, is that the probiotic strains are significantly more stable at pH3 than proprietary milk-based or freeze-dried probiotic products, and therefore more likely to be capable of tolerating the harsh conditions encountered in the human GI tract. In particular, it has been shown that the probiotic bacteria in the preparation described herein are stable both at pH6 and at pH3. In exemplary embodiments the probiotic bacteria in the probiotic preparation are stable when maintained in culture at pH 3 for a period of at least 6 hours. In this context "stable" can be taken to mean that when the bacteria are cultured at pH 3, 37°C, in standard culture medium (e.g. MRS broth) then over a period of at least 6 hours the bacterial count (cfu/ml) does not fall by more than 0.5 log₁₀ units below the bacterial count (cfu/ml) at time zero. In specific embodiments, the bacterial count should remain above 10⁶ cfu/ml for at least 6 hours when cultured at pH 3, 37°C, in standard MRS broth. In fact, the bacterial count may be observed to increase when cultured under these conditions.

### Composition of the probiotic preparation

The probiotic preparation described herein and shown to be clinically effective in the treatment of IBS comprises viable, metabolically active probiotic bacteria in a non-dairy liquid substrate. The function of the substrate is to support, and maintain viability of, the probiotic bacteria, particularly during storage of the preparation, such that the probiotic bacteria can be maintained, and then delivered to the patient, in a viable, metabolically active form. To achieve this objective, the liquid substrate typically contains a mixture of complex carbohydrates and simple sugars. In particular, the substrate may comprise a mixture of polysaccharides, oligosaccharides, disaccharides and monosaccharides.

In certain embodiments, the probiotic preparation may be characterised by the ratio of total carbohydrate content to reducing sugar content of the preparation, reflecting the complex mix of polysaccharides, oligosaccharides, disaccharides and monosaccharides present. In exemplary embodiments, the ratio of total carbohydrate content to reducing sugar content of the preparation is in the range of from 8:1 to 2:1, more typically in the range of from 5:1 to 2.5:1, or in the range of from 4:1 to 3:1. Means of measuring the total carbohydrate content and reducing sugar content, and calculating the ratio between them, are as defined above.

In further exemplary embodiments of the probiotic preparation, the total carbohydrate content of the preparation may be in the range of from 20 mg/ml to 40 mg/ml, or in the range of from 20 mg/ml to 30 mg/ml and the total reducing sugar content may be in the range of from 5 mg/ml to 20 mg/ml, or in the range of from 5 mg/mg to 10 mg/ml.

The probiotic preparation preferably also comprises protein and peptide components. Typically the total amount of protein and peptides present in the probiotic preparation is in the range of from 1 mg/ml to 2 mg/ml and the total amount of high molecular weight peptides (molecular weight greater than 5000 Daltons) is in the range of from 100 µg/ml to 300 µg/ml. In a specific embodiment, the concentration of protein and peptides may be about 2mg/ml and the concentration of high molecular weight peptides may be about 250µg/ml.

The probiotic preparation may contain further components such as, for example, cellulose, starch, β-glucans, pentosans, polyphenols, ribonucleic acids, lipids, phosphates, flavenoids, amino acids, vitamins (B₁, B₂, C and E), silicates and trace elements.

The probiotic preparation may comprise an extract of germinated barley containing the desired probiotic bacterial strains.

An exemplary embodiment of the probiotic preparation comprises extract of germinated barley and a combination of *Enterococcus faecium, Lactobacillus plantarum,* and either *Lactobacillus casei* or *Lactobacillus rhamnosus* or both *Lactobacillus casei* and *Lactobacillus rhamnosus,* wherein the bacterial count for each of these bacterial strain is in the range of from 1.0 x 10⁵ to 1.0 x 10⁹ viable cells per millilitre, more preferably in the range of from 1.0 x 10⁷ to 1.0 x 10⁹ viable cells per millilitre, and further contains *Lactobacillus acidophilus.*

Additional components may be added to the probiotic preparation, such as flavourings and/or colourings, to improve palatability for human patients.

The pH of the preparation can be conveniently controlled by the addition of a suitable buffer or combination of buffering agents. Preferred buffers include, for example, tri-sodium citrate or phosphate buffers. The pH of the liquid-based preparation described herein is typically maintained in the range of from 3.8 to 4.5, and in particular at about pH 4.0, during long-term storage. The probiotic preparation may be stored at any temperature from 4°C up to ambient temperature (about 25°C). The Symprove™ product described herein has been shown to remain stable (in terms of bacterial count) for a period of at least 6 months when stored at about 4°C, and for at least 4 months when stored at 25°C.

The preparation may additionally comprises an anti-fungal agent, such as, for example, sterilised potassium sorbate and/or and anti oxidant, such as vitamin C.

In a preferred embodiment the growth substrate may contain particulate matter, for example particles not exceeding 1mm in diameter.

The most preferred embodiment of the probiotic preparation, shown to be effective in the treatment of IBS in the clinical study described herein, is the product denoted Symprove™, containing viable, metabolically active cells of *Enterococcus faecium, Lactobacillus plantarum*, *Lactobacillus casei* and *Lactobacillus acidophilus,* which may be prepared according to the examples provided herein. The strain of *Lactobacillus casei* in Symprove™ was originally characterised as *Lactobacillus casei* but has now been re-classified as the closely related *Lactobacillus rhamnosus.*

The Symprove™ product is water-based, with a typical water content of about 95% (v/v). This high water content is advantageous since it may result in the product being perceived by the human GI system as a drink rather than "food", thus avoiding triggering digestion.

The probiotic preparation may also be lactose-free, since it is not milk-based, and gluten-free. In particular the Symprove™ product is demonstrated to fulfil the accepted criteria for classification as "gluten-free" based on Gliadin ELISA. Batches of the product typically contain less than 10ppm gluten when measured by Gliadin ELISA.

### Treatment of IBS

Preferably, the treatment of Irritable Bowel Syndrome comprises amelioration of the symptoms associated with the conditions. Thus, treatment of IBS may include amelioration of one or more of stomach pain, stomach cramps, diarrhoea, constipation and bloating. Treatment of IBS includes treatment of both constipation-dominant and diarrhoea-dominant forms of the condition. As demonstrated herein, treatment of patients with established IBS, including individuals with a diagnosis according to the Rome III criteria who have failed first and second line treatment, may result in a significant improvement in IBS symptom severity.

The precise dosage will depend on many factors, not least the severity of an individual patient's condition. The most appropriate dose can be determined by a medical practitioner and generally should be a dose which results in a beneficial effect, e.g. improvement in INS symptom severity. Typically dosing of the Symprove™ product prepared according to the examples could be 1ml/kg body weight per day or 50ml per day for an adult patient. The frequency and duration of treatment will vary from patient to patient. A typical treatment period is 12 weeks, but the period may be longer or shorter. Typically the dosage frequency would be daily, although other dosage frequencies may also be used.

In a preferred embodiment, the Irritable Bowel Syndrome is Rome III classified Irritable Bowel Syndrome. In another preferred embodiment, the patient in need of treatment is a patient who has failed first and second line treatments.

For treatment of human patients with IBS, the liquid probiotic preparation is typically administered orally, e.g. as a drink. If convenient, the probiotic preparation may be incorporated into foodstuffs or beverages for human consumption, provided that this does not affect the viability of the bacteria in the preparation.

For human use, in the treatment of IBS, it may be convenient to formulate the probiotic preparation into unit dosage form, e.g. a single dose aliquot of the liquid preparation which is ready-to-drink.

The probiotic preparation to be used in the treatment of IBS as described herein is typically a liquid-based preparation which is advantageously not freeze dried.

Preparations of freeze-dried bacteria which are currently used typically contain few, if any, metabolically active bacteria. Furthermore, preparations of freeze-dried bacteria lose some of their characteristics and activity upon rehydration. Also, the reactivated bacteria have a short shelf life and can be kept in storage for a short time only. Freeze-dried bacteria are often not rehydrated before use in a host animal. If they are rehydrated, then they generally have to be used on the same day, otherwise there may be rapid loss in viability and spoilage from contaminating organisms.

A further problem with prior art freeze-dried preparations is that they are hygroscopic (attract moisture from the air) and therefore a packet has to be used within a few days of opening, otherwise the partial hydration will cause rapid loss of viability.

The liquid-based probiotic preparation provided herein for use in treating IBS avoids the above-listed problems associated with the use of freeze-dried bacterial cultures. In addition, it has also been shown that the liquid-based bacterial cultures prepared as described herein are significantly more robust than bacteria prepared by methods known in the prior art, which enables the bacteria to establish more rapidly in host animals and to tolerate the harsh environment of the mammalian digestive tract.

### Manufacture of the probiotic preparation

The probiotic preparation for use in treatment of IBS as described herein may be prepared by growing one or more probiotic bacterial strains in a liquid growth substrate, such as for example an extract of germinated barley. The growth substrate may be itself prepared starting from seed or malting sample barley using the manufacturing process described hereinbelow. This method is substantially as described in WO 2006/035218.

The growth substrate may be prepared according to a method comprising:
subjecting malted cereal to a mashing step in which the malted cereal is mixed with aqueous liquid and subjected to conditions of time and temperature which limit the extent of conversion of complex carbohydrates to simple sugars such that a mixture of complex carbohydrates, simple sugars, proteins and peptides is obtained; and
separating the mixture of complex carbohydrates and simple sugars, proteins and peptides from the spent malted cereal to obtain a growth substrate.

The terms "malted cereal" or "malt" as used herein refers to the product of a malting process applied to cereal grains. Malting is a process well known in the art of brewing.

In a typical malting process cereal grains (e.g. seed or malting sample barley) are germinated to induce the mobilization of storage nutrients. Germinating seeds produce a number of enzymes to mobilise storage proteins and carbohydrates, including α-amylases which hydrolyse starch into maltose. An exemplary cereal is barley, but other cereals, such as rice, wheat, corn and oats or even mixtures thereof, may also be used. The process of germination is generally well known. It will be appreciated that the method may be carried out by providing malted grains or a synthetic substrate comprising a mixture of carbohydrates, proteins and enzymes.

Typically the malting grains are rolled before further processing. Cracking of the grains by rolling facilitates access of water and extraction of nutrients during the mashing step, whilst avoiding shattering of the grains assists in the subsequent separation of the growth substrate from spent malted grains.

The prepared malt is subjected to a mashing step which resembles the mashing-in step used in methods for brewing (see, for example, Kunze, W. Technology Brewing and Malting (1996)). The term "mashing-in" is well known in the field of brewing and relates to a process wherein malted grains are agitated in the presence of water heated to defined temperatures in order to prepare a wort. During the mashing in step complex carbohydrates in the malted grains are broken down into maltose.

The method described herein also involves a mash step in which malted cereal grains are mixed with an aqueous liquid (typically water) and the mixture heated to various defined temperatures. This mash step does not, however, conform to a typical brewer's mashing-in process. Brewers aim to convert as much carbohydrate to simple sugars as possible, for subsequent fermentation to alcohol. In contrast, the conditions of the mash step described herein are specifically chosen to limit the extent of conversion to simple sugars, leaving significant amounts of carbohydrates in more complex oligomeric and polymeric forms.

The extent of conversion of carbohydrate may be limited such that the amount of reducing sugars present in the resulting growth substrate, expressed as a percentage (w/w) of total carbohydrate content, is in the range of from 10% (w/w) to 50% (w/w). As noted above, the reducing sugar content measured by the Nelson-Somogyi method is a good approximation of the total amount of simple sugars present.

The desired limited conversion of complex to simple sugars may be achieved by increasing the temperature in the mash step over a short period of time, typically 30 minutes, without allowing the mixture of malted grains/water to rest at intermediate temperatures. Traditional brewing processes include rests at 60-65°C and 70-74°C, as this allows enzymes to produce high concentrations of simple sugars (mainly maltose). Accordingly, the mashing-in step described herein does not comprise rests at temperatures in the range of from 60°C to 65°C and/or at temperatures in the range of from 70°C to 74°C.

The mash step may comprise mixing the malted cereal with water at a temperature in the range of from 30°C to 45°C, resting the mixture for 1 to 2 hours, increasing the temperature to a temperature in the range of from 75°C to 85°C over a time period in the range of from 20 to 60 minutes, preferably 30 minutes, and then resting the mixture at a temperature in the range of from 75°C to 85°C for a period of time in the range of from 30 to 90 minutes. At the higher temperature, any enzymes present in the mixture are inactivated and nutrients can be extracted. Higher temperatures in the range of from 76°C to 80°C and specifically 78°C are generally preferred. The temperature in this step needs to be sufficiently high for sufficiently long to inactivate all enzymes present in the preparation. It will be appreciated that the precise temperature and times used can vary somewhat according to the type of cereal used.

The process described here specifically limits the amount of conversion of complex sugars to simple sugars. Furthermore, the initial rest at a temperature in the range of from 30°C to 45°C provides an additional advantage in that it maximises the release of amino acids and peptides. Temperatures towards the higher end of this range, i.e. from 40°C to 45°C or specifically about 45°C, are generally preferred. The purpose of this step is to hydrolyse storage proteins present in the cereal grains into available amino acids and peptides. The optimum combination of time and temperature to achieve the desired hydrolysis may vary somewhat depending on the type of grains used.

The presence of high concentrations of proteins, peptides and amino acids is desirable in a growth substrate to be used to support the growth of microorganisms as it provides a useful a source of nitrogen. Typical mashing-in processes used in traditional brewing would generally not include a rest at a temperature in this range, since brewers do not normally seek to achieve high protein or amino acid content in a wort intended for fermentation to produce alcohol.

When the mash step is complete, e.g. all the desired nutrients have been extracted from the now "spent" malted grains, the resulting mixture comprising complex carbohydrates and simple sugars, proteins and peptides may be separated from the spent grains to obtain a growth substrate using any suitable means. Typically this will involve coarse filtration, for example using a 1mm filter such as a wedge-wire basket, yielding a solution containing coarse particles. It is a feature of the process described herein that the growth substrate is not clarified, as would generally be the case with a wort prepared during standard brewing. In traditional brewing the wort is generally clarified to remove all coarse particles, thereby producing a clear liquid.

The presence of some particulate matter in a growth substrate prepared according to the process described herein is advantageous as regards subsequent use in supporting the growth of bacteria since it provides both slow release nutrients and particulate surfaces for the adhesion of bacterial cultures.

If required, the growth substrate prepared according to the process described herein can be sterilised prior to further usage. As will be appreciated, this can be carried out by boiling for about one hour or by autoclaving at 120°C for about 20 minutes.

A buffer, or several buffering components may be added to the growth substrate if required. Preferred buffers are tri-sodium citrate or phosphate buffers. If the growth substrate is to be sterilised, the buffer(s) may be added prior to, during or after sterilisation as convenient.

The prepared growth substrate is then inoculated with the desired probiotic bacterial strains and grown until they reach the desired bacterial count required in the probiotic preparation. In a specific embodiment, the probiotic bacteria are grown in the growth substrate until they reach a concentration of between 1 x 10⁶ and 1 x 10⁹ colony forming units per millilitre. At this point the culture (or fermentation) can be cooled down to about 4°C and the combination of temperature, pH, phase of growth and residual substrate composition provides equilibrium conditions which allows for maintenance of a near equilibrium growth state during long term storage. In this near equilibrium state, which can be maintained for a period of at least 5-6 months, the population of metabolically active probiotic bacteria is maintained, typically in the range of from 10⁶ to 10⁹ viable cells per millilitre. It will be appreciated that the precise number of viable cells at the near equilibrium state can vary somewhat depending on the species of probiotic bacteria used. Typically the bacterial count (cfu/ml) is "stable" meaning that it does not vary by more than 1 log₁₀ unit, or more preferably by more than 0.5 log₁₀ unit, when the product is stored at 4°C for 5 months and/or when stored at 25°C for 4 months.

This manufacturing method involves a growing step in which the probiotic bacteria are grown in a growth substrate until they reach a concentration which enables the near-equilibrium condition to be achieved during subsequent storage. It is an important feature of the method that the growth substrate used provides a balanced nutrient supply containing a mixture of complex carbohydrates and simple sugars wherein a high proportion of carbohydrate content is in the form of complex carbohydrates that may be used as an energy source by the probiotic bacteria. This mix helps to limit immediately available energy during the growing step. The composition and preferred features of the growth substrate is/are preferably as described above. The growth substrate is preferably prepared from malted cereal grains using the manufacturing method described herein, and is typically an extract of germinated barley. It will be appreciated, however, that it is not strictly necessary to use growth substrate prepared according to this method. Similar results can be achieved using growth substrate prepared synthetically by mixing the required proportions of complex carbohydrates and simple sugars, proteins and peptides.

The growing step must be carried out in such a way that care is taken not to grow the probiotic bacteria for too long, to avoid producing acid conditions which would otherwise inhibit further growth as well as limiting the period of storage of the resulting preparation. On the other hand, if the growing step is terminated prematurely, this will limit biomass production. The pH of the preparation during growth of the probiotic bacteria can be used as an indicator of the near equilibrium state. In a typical embodiment, cultures of lactic acid bacteria may be grown until a pH of 4.5 +/- 0.3 units is reached.

It will be appreciated that the pH of the growth substrate may vary somewhat depends on the optimal pH range for the probiotic bacteria used. In a typical embodiment (suitable for lactic acid bacteria) pH should be maintained in the range of from 3.8 to 4.5 during storage. Preferably buffers, such as tri-sodium citrate or phosphates are added to the growth substrate to control the pH during growth of the probiotic bacteria and subsequent storage.

It will be appreciated that the exact timing of the step of growing the probiotic bacteria to a near equilibrium state depends on the species used. Growth of the probiotic bacteria can be carried out in any suitable culture apparatus. In specific embodiments the growing step can be carried out by way of fermentation in a fermentation vessel. The probiotic bacteria used are lactic acid bacteria of the genera *Enterococcus* and *Lactobacillus,* and mixtures thereof, as described above. If such species were to be grown using "conventional" growth media consisting largely of fermentable simple sugars the excess energy supply would lead to excess acid production by lactic acid bacteria, which would limit biomass production and shelf life of the resulting culture. In contrast, the mix of complex carbohydrates and simple sugars present in the growth substrate used in the manufacturing method described herein supplies energy for growth in the initial growing step and also for maintenance during storage of the product.

The growing step can be carried out using a single species of probiotic bacteria, and two or more different bacterial species may be grown independently in separate growth media and then blended together before or after the chilling step. The growth substrates used for independent culture of two or more different bacterial species may be the same or of different composition. Thus, it is possible to optimise the growing conditions for cultures of individual bacterial species and then combine the cultures together for storage under conditions which permit maintenance of equilibrium growth for each of the individual species in the culture.

In other manufacturing methods, two or more different bacterial species can be grown together in a single culture in the same growth substrate, provided that their growth rates are comparable so that one species does not dominate the population.

Following the growing step, the probiotic preparation can be analysed by standard methods to determine the microbiological purity and enumeration of probiotic bacteria. It is also possible to grow two or more different combinations of probiotic bacteria together in separate growth substrates and then combine the cultures together in a final probiotic preparation. A combination culture could similarly be blended with a culture of a single bacterial species.

Starter cultures for the growing step of the method include, for example, freeze-dried bacteria or liquid cultures.

It will be appreciated that the probiotic preparation may be used immediately but more typically the preparation will be stored before use. The optimum temperature for storage in order to maintain the near equilibrium growth state in the culture is about 4°C but it will be appreciated by the skilled reader that the storage temperature could vary somewhat. As noted above, the probiotic preparation is stable fro at least 4 months even when stored at ambient temperature (about 25°C). For convenience, aliquots of the probiotic preparation produced by the method may be dispensed into suitable sterile packaging prior to long term storage.

It is a key advantage of the probiotic preparation described herein that the product maintains viability and integrity [of the probiotic bacteria] when stored for extended periods, typically for at least 5 or 6 months. However, it will be appreciated that it is not essential to the method that the probiotic preparation must actually be stored for 5-6 months prior to use.

To avoid the growth of fungi or yeast, an anti fungal agent, such as sterile potassium sorbate, may be added prior to storage of the probiotic preparation. The anti fungal agents are primarily to prevent spoilage by yeast or fungi during use by end-users, once a container of the product has been opened. Furthermore, an anti oxidant, for example, vitamin C can be added to help to prevent spoilage of the product during storage. It will be appreciated that other agents well known in the art can also be used as anti fungal agents or anti oxidants.

An alternative method of producing the probiotic preparation which does not require an active growing step simply involves inoculated the growth substrate (e.g. the extract of germinated barley prepared as describe herein) with starter culture(s) of probiotic bacteria. Starter cultures for the method include, for example, freeze-dried bacteria or liquid cultures. The growth substrate may be inoculated with more than one bacterial species. Preferably, the concentration of the viable cells in the starter culture is in excess of 10⁶ viable cells per millilitre.

The invention will be further understood with reference to the following non-limiting experimental examples:

### Example 1: Production of a growth substrate, using a barley-based medium.

### (A) Germination (malting)

### Day 1

Barley (seed or malting sample grains) was steeped in water for 2 to 24 hours, depending on the batch of grain. 0.1% (w/v) sodium hypochlorite (bleach) can be included in the water to inhibit growth of contaminants during the germination phase. After 4 hours, the water was drained from the grains and the grains left to stand at room temperature from 10 to 30°C for approximately 1 day.

### Day 2

The grains were steeped in clean water for another 4-hour period. Hydrogen peroxide (0.1% w/v) may be added to the water for this and subsequent soaks. Hydrogen peroxide provides oxygen for the germinating grains, and act as a disinfectant. Alternatively, sodium hypochlorite may be used. After 4 hours, water was drained from the grains and the grains left to stand for approximately 1 day. Occasional (e.g. every 4 hours) agitation of the grains may improve germination by increasing gaseous exchange, providing oxygen and removing carbon dioxide.

### Day 3 onwards

The cycle of steeping, draining and standing was continued until the emerging rootlets on the germinating grains were 2 to 4 mm long. This state of growth indicated that the grains had produced enzymes for the mobilisation of stored nutrients. These enzymes are central to the subsequent production of the growth medium, during 'mashing-in.' More extensive modification of the grains could be allowed, leaving the germination phase until the rootlets are several millimetres long. However, too much growth will merely convert nutrients into plant roots and shoots, which cannot be used in the fermentation.

### (B) Rolling

When the grains had germinated sufficiently, they were then milled with a roller mill. The mill was adjusted such that the grains were cracked open but not shattered or completely flattened. Cracking of the grains allows access of water and extraction of nutrients during mashing in, whilst avoiding shattering of the grains assists in filtration steps.

### (C) Preparation of a growth substrate.

The germinated, milled, grains were mixed with sufficient water to cover them, at 45°C, and the mixture held at 45°C for 1 hour.

After 1 hour at 45°C, the temperature was increased to 78° C over a period of 30 minutes.

The mixture was allowed to stand at 78°C for 1 hour. After the 78°C stand, the spent grains were separated out by filtration. A relatively coarse filter (e.g. 1mm gap wedge-wire basket) was used yielding a solution containing significant amounts of suspended solids. The spent grains were discarded and the liquid then heat-treated to pasteurise or sterilise it. In this particular experiment the liquid was boiled for 45 minutes. A buffer (0.5% (w/v) tri-sodium citrate) was then added and the mixture boiled for a further 15 minutes to yield the final growth substrate.

### Example 2: Analysis of the growth substrate

### (a) Carbohydrate Analyses:

Total carbohydrate content was determined using the phenol-sulphuric acid assay, with glucose as reference standard. (Dubois, M., Gilles, K. A., Hamilton, J. K., Rebers, P. A. and Smith, F. (1956) Analytical Chemistry, vol. 28., p. 350).

Reducing sugars were determined using the Nelson-Somogyi method, again with glucose as the reference standard. (Somogyi, M. (1952) Journal of Biological Chemistry., vol. 195., p. 19). Both complex and simple sugars have reducing ends, but simple sugars have a greater number of reducing ends per unit mass. Thus, whilst the Nelson-Somogyi method does detect complex sugars, the signal is so low so as to be negligible. The Nelson-Somogyi method therefore gives a good approximation of the level of simple sugars in a sample.

### Results:

Total sugars in substrate are in the range 20 to 40 mg/ml (milligrams per millilitre)

Reducing sugars are in the range 5 to 20 mg/ml.

### (b) Protein and Peptide Analyses:

Total protein was determined using two assays, with bovine serum albumin as reference standard:
(i) The Biuret Reagent (Itzhaki, R. F & Gill, D. M. (1964) Analytical Biochemistry, Vol. 9., p. 401-410.
(ii) The Lowry Method, as modified by Ohnishi and Barr. (Ohnishi, S. T. & Barr, J. K. (1978) Journal of Biological Chemistry, Vol. 193, p. 265).

Peptides of molecular weight above 5000 daltons were determined using the Bradford Reagent (Bradford, M. M. (1976) Analytical Biochemistry, Vol. 72, p. 248-254).

### Results:

Total protein and peptides are in the range 1 to 2 milligrams per millilitre.

High molecular weight peptides (greater than 5000 daltons) are in the range 100 to 300 micrograms per millilitre.

### Example 3: Production of a metabolically active bacterial culture

### (A) Fermentation

The growth substrate prepared according to example 1 was cooled to 37°C and the bacterial cultures added. Examples of a suitable inoculum are freeze-dried bacteria or liquid starter cultures (typically 1% (v/v) of an overnight culture in nutrient broth).

In this example the following bacteria were grown in two vessels:
(i) *Enterococcus faecium, Lactobacillus plantarum;*
*(ii)* A strain of *Lactobacillus casei* which has now been re-classified as *Lactobacillus rhamnosus, Lactobacillus acidophilus*

Fermentation was carried out for 16-20 hours, until the pH reached 4.5 +/- 0.3 units. The fermentation mixture was then cooled to 4°C and subjected standard techniques to assess quality (to determine microbiological purity and enumeration of bacteria).

At this point, sterile potassium sorbate (0.005% w/v final concentration) may be added to the fermented broth. This acts to inhibit the growth of any fungi or yeast that may arise due to contamination during handling by the end-user. Vitamin C may also be added (0.01% w/v final concentration) as an anti-oxidant.

Following quality assurance tests, different batches can be blended to give products with complex mixtures of bacterial species, if required.

In the example presented here, blending of the two batches of bacteria yielded a final product containing the bacteria *Enterococcus faecium*, *Lactobacillus plantarum, Lactobacillus acidophilus* and the strain of *Lactobacillus casei* which has now been re-classified as *Lactobacillus rhamnosus.*

Batches of this final product typically contain 2.3g carbohydrate per 100ml, of which 0.7g is sugars and 1.6g is starch. The bacterial count in the final product is typically less than 1.0 x 10⁵ for L. *acidophilus* and typically at least 1.0 x 10⁷ for each of, L. *plantarum*, E. *faecium* and the strain of *Lactobacillus casei* which has now been re-classified as *Lactobacillus rhamnosus.*

Batches of the final product were tested for the presence of gluten using the Gliadin ELISA test and were determined to contain less than 15ppm gluten, and more typically less than 10ppm gluten, satisfying the Codex Alimentarius definition of gluten-free.

The Symprove™ product described herein is commercially available from Multigerm UK Enterprises Ltd.

### Example 4: Beneficial Effects of the preparation in the treatment of Irritable Bowel Syndrome

A study of 186 patients with Rome III classified IBS, showed that a product prepared according to example 3 (referred to herein as Symprove™, commercially available from Multigerm UK Enterprises Ltd), comprising a mixture of *Enterococcus faecium, Lactobacillus acidophilus*, *Lactobacillus plantarum* and a strain of *Lactobacillus casei* which has now been re-classified as *Lactobacillus rhamnosus,* can improve even the most severe IBS symptoms.

This was a single centre, double blind, randomised, placebo controlled trial involving treatment with Symprove™ (1 ml/kg body weight) versus placebo for 3 months. Patients were recruited from two large GP practices in London and from GP referrals to the Department of Gastroenterology at King's College Hospital, London. All patients had failed conventional first and second line treatment by GPs. An initial 391 patients were screened for the trial and 186 were eligible and consented to the trial (fulfilled ROME III criteria, exclusion of organic disease by extensive investigation including intestinal permeability, faecal calprotectin, endoscopy, wireless capsule endoscopy, colonoscopy and radiology when indicated). Using a simple non-stratified two-stage computer randomisation protocol (the Mersenne twister pseudo-random number generator) 124 received the active treatment and 62 placebo. The main outcome measure was the reduction in IBS symptom severity scores (IBS-SSS) at 3 months. All patients were symptomatic with IBS-SSS > 150.

There were no significant differences between the two treatment groups with respect to demographic details (male/female ratio, mean age, duration of disease or previous treatment). There were no significant differences (p > 0.1) at pre treatment IBS-SSS scores between active (303 ± 68 mean ±SD) and placebo (306 ±80) (p=0.841) treated patients. During the last week of treatment (week 12) there was a statistically significant (p < 0.027) greater improvement in the mean IBS-SSS (230, ± 109) in the Symprove™ treated patients as compared with placebo (270±103). The number of dropouts during the study did not differ significantly between the 2 groups and no severe adverse events were evident.

The data gathered from this double blind, investigator-led study using a masked comparator, shows a statistically significant improvement in IBS symptom severity (pre-trial versus the last two weeks of treatment; measured on the IBS symptom severity index) with the product prepared according to example 3 (referred to herein as Symprove™) when compared to placebo (p=0.012). The study was more robust than most other probiotic studies in IBS, and involved a 3 month treatment period in IBS patients who had failed first and second line treatments and where symptoms were sufficiently severe for GP's to refer to hospital. These patients constitute the top 1% in terms of symptom severity.

The success of this trial indicates that the underlying pathology of IBS - whatever its exact nature - is being addressed by treatment with the product of example 3.

### Example 5: Comparison of the product of example 3 with other probiotics at physiological pH

A proprietary multi-strain freeze-dried probiotic product (referred to herein as MS-FD), a popular single strain milk-based probiotic product(referred to herein as SS-MB) and the final product of example 3 (identified herein as Symprove™, a multiple strain probiotic containing viable, metabolically active bacterial cells) were compared by inoculating an MRS broth of known pH maintained at 37°C with each product according to the manufacturers instructions. Aliquots of each were then plated onto agar gel every 30 minutes for six hours, incubated and cfu counts performed.

At pH6 both multiple strain probiotics quickly achieved stable cfu counts, MS-FD peaking at 2.2 x 10⁸ and Symprove™ reaching 1.1 x 10⁹ cfus over six hours, the single strain probiotic (SS-MB) yielded a lower count with at most 2.4 x 10⁶ detectable cfus over the 6 hours. At pH3 both MS-FD and SS-MB exhibited rapidly decreasing cfu counts at each successive 30 minute data point, both dropping below 1000 cfus at 6 hours. The Symprove™ product maintained stable cfu counts above 10⁶ at all data points with an increase in cfus becoming apparent after 4 hours and reaching 4.8 x 10⁷ at six hours. The results are displayed in figures 1 and 2.

In this model the Symprove™ product was the only probiotic to demonstrate the ability to deliver consistent cfu counts immediately after, and for six hours following, inoculation at two physiological pH levels. In this experiment the Symprove™ product delivered the highest final cfu count at both pH3 and pH6.

### References

F. Fonseca, C. Béal, and G. Corrieu. J.Dairy Res. 67 (1):83-90, 2000.
M. L. F. Murga, A. P. D. Holgado, and G. F. de Valdez. Cryobiology 36 (4):315-319, 1998.
J. Hamilton-Miller. Lancet 355 (9201):413-414, 2000.
Kunze, W. Technology Brewing and Malting (1996)

### Diarrhoea and Constipation

T. Arvola, K. Laiho, S. Torkkeli, H. Mykkänen, S. Salminen, L. Maunula, and E. Isolauri. Pediatrics 104 (5):L1-L4, 1999.
R. Bennet, S. L. Gorbach, B. R. Goldin, T. W. Chang, B. E. Laughon, W. B. Greenough, and J. G. Bartlett. Nutrition Today 31 (6):35S-38S, 1996.
A. Bomba, R. Nemcová, S. Gancarcíková, R. Herich, and R. Kastel. Adv.Exp.Med.Biol. 473:185-190, 1999.
N. M. De Roos and M. B. Katan. Am.J.Clim.Nutr. 71 (2):405-411, 2000.
H. L. DuPont. J.Pediatr. 134 (1):1-2, 1999.
S. L. Gorbach. Am.J.Gastroenterol. 95 (1):S2-S4, 2000.
M. Heyman. J.Am.Coll.Nutr. 19 (2):137S-146S, 2000.
E. Isolauri, M. Kaila, H. Mykkanan, W. H. Ling, and S. Salminen. Dig.Dis.Sci. 39 (12):2595-2600, 1994.
R. A. Oberhelman, E. H. Gilman, P. Sheen, D. N. Taylor, R. E. Black, L. Cabrera, A. G. Lescano, R. Meza, and G. Madico. J.Pediatr. 134 (1):15-20, 1999.
R. D. Rolfe. J.Nutr. 130 (2):396S-402S, 2000.
J. Saavedra. Am.J.Gastroenterol. 95 (1):S16-S18, 2000.
C. Scarpignato and P. Rampal. Chemotherapy 41 ((suppl 1)):48-81, 1995.
A. V. Shornikova, I. A. Casas, H. Mykkänen, E. Salo, and T. Vesikari. Pediatr.Infect.Dis.J. 16 (12):1103-1107, 1997.
J. A. Vanderhoof, D. B. Whitney, D. L. Antonson, T. L. Hanner, J. V. Lupo, and R. J. Young. J.Pediatr. 135 (5):564-568, 1999.

### Irritable Bowel Syndrome

P. Brigidi, B. Vitali, E. Swennen, G. Bazzocchi, and D. Matteuzzi. Res.Microbiol. 152 (8):735-741, 2001.
K. Niedzielin, H. Kordecki, and B. Birkenfeld. Eur.J.Gastroenterol.Hepatol. 13 (10):1143-1147, 2001.
S. Nobaek, M. L. Johansson, G. Molin, S. Ahrne, and B. Jeppsson. Am.J.Gastroenterol. 95 (5):1231-1238, 2000.
WO2007/036230

### General review:

Marteau, P. and Rambaud, J-C, (1996) 'Therapeutic applications of probiotics in humans' in Leeds, A. R. and Rowland, I. R. (eds.) Gut flora and health - past, present and future, Royal Society of Medicine Press Limited, London. Stark, B. A. and Wilkinson, (eds.) (1989) Probiotics. Theory and Applications, Chalcombe Publications, Bucks, UK. 21

### Probiotic compositions:

WO2006/035218
WO2011/078781

## Claims

1. A probiotic preparation comprising viable, metabolically active probiotic bacteria in a non-dairy liquid substrate, for use in the treatment of Irritable Bowel Syndrome, wherein the probiotic preparation comprises a mixture of *Enterococcus faecium, Lactobacillus plantarum, Lactobacillus acidophilus,* and either *Lactobacillus casei* or *Lactobacillus rhamnosus* or both *Lactobacillus casei* and *Lactobacillus rhamnosus.*

2. The probiotic preparation for use according to claim 1, wherein the probiotic preparation comprises a mixture of complex carbohydrates and simple sugars, wherein the ratio of total carbohydrate content to reducing sugar content of the probiotic preparation is in the range of from 8:1 to 2:1.

3. The probiotic preparation for use according to any one of the preceding claims, wherein the total count of metabolically active bacteria in the preparation is in the range of from 1.0 x 10⁶ to 1.0 x 10⁹ viable cells per millilitre.

4. The probiotic preparation for use according to claim 1 wherein the total count of at least one of, and more preferably each of *Enterococcus faecium, Lactobacillus plantarum,* and either *Lactobacillus casei* or *Lactobacillus rhamnosus* or a combination of *Lactobacillus casei* and *Lactobacillus rhamnosus* in the preparation is at least 1.0 x 10⁶ viable cells per millilitre.

5. The probiotic preparation for use according to claim 1 wherein the total count of at least one of, and more preferably each of *Enterococcus faecium, Lactobacillus plantarum,* and either *Lactobacillus casei* or *Lactobacillus rhamnosus* or a combination of *Lactobacillus casei* and *Lactobacillus rhamnosus* in the preparation is at least 1.0 x 10⁷ viable cells per millilitre.

6. The probiotic preparation for use according to any one of the preceding claims, wherein the probiotic preparation has a total carbohydrate content in the range of from 20 mg/ml to 40 mg/ml and a reducing sugar content in the range of from 5 mg/ml to 20 mg/ml.

7. The probiotic preparation for use according to any one of the preceding claims which additionally comprises proteins and peptides, wherein the total amount of protein and peptides is in the range of from 1 mg/ml to 2 mg/ml and wherein the total amount of high molecular weight peptides is in the range of from 100 µg/ml to 300 µg/ml.

8. The probiotic preparation for use according to any one of the preceding claims wherein the pH of the preparation is maintained in the range of from 3.8 to 4.5.

9. The probiotic preparation for use according to any of the preceding claims wherein the probiotic preparation is prepared by growing the probiotic bacterial strain(s) in an extract of germinated barley.

10. The probiotic preparation for use according to any one of the preceding claims wherein the preparation further comprises an anti-fungal agent preferably wherein the anti-fungal agent is sterilised potassium sorbate.

11. The probiotic preparation for use according to any one of the preceding claims wherein the preparation further comprises an anti-oxidant preferably wherein the anti-oxidant is vitamin C.

12. The probiotic preparation for use according to any preceding claim, for use in the treatment of patients diagnosed with Rome III classified Irritable Bowel Syndrome.

13. The probiotic preparation for use according to any preceding claim, for use in the treatment of patients diagnosed with Irritable Bowel Syndrome who have failed first and second line treatments for Irritable Bowel Syndrome.

14. The probiotic preparation for use according to any preceding claim, for the treatment of patients with Irritable Bowel Syndrome symptom severity score of greater than 150.

## Patentansprüche

1. Probiotische Zubereitung umfassend lebensfähige, metabolisch aktive, probiotische Bakterien in einem milchfreien, flüssigen Substrat zur Verwendung bei der Behandlung von Reizdarmsyndrom, wobei die probiotische Zubereitung eine Mischung von *Enterococcus faecium, Lactobacillus plantarum, Lactobacillus acidophilus* und entweder *Lactobacillus casei* oder *Lactobacillus rhamnosus* oder sowohl *Lactobacillus casei* als auch *Lactobacillus rhamnosus* umfasst.

2. Probiotische Zubereitung zur Verwendung nach Anspruch 1, wobei die probiotische Zubereitung eine Mischung von komplexen Kohlenhydraten und einfachen Zuckern umfasst, wobei das Verhältnis von Gesamtkohlenhydratgehalt zu Gehalt an reduzierendem Zucker der probiotischen Zubereitung im Bereich von 8:1 bis 2:1 ist.

3. Probiotische Zubereitung zur Verwendung nach einem der vorhergehenden Ansprüche, wobei die Gesamtzahl an metabolisch aktiven Bakterien in der Zubereitung im Bereich von 1,0 x 10⁶ bis 1,0 x 10⁹ lebensfähige Zellen pro Milliliter ist.

4. Probiotische Zubereitung zur Verwendung nach Anspruch 1, wobei die Gesamtzahl von wenigstens einem und stärker bevorzugt von jedem von *Enterococcus faecium, Lactobacillus plantarum* und entweder *Lactobacillus casei* oder *Lactobacillus rhamnosus* oder einer Kombination von *Lactobacillus casei* und *Lactobacillus rhamnosus* in der Zubereitung wenigstens 1,0 x 10⁶ lebensfähige Zellen pro Milliliter ist.

5. Probiotische Zubereitung zur Verwendung nach Anspruch 1, wobei die Gesamtzahl von wenigstens einem und stärker bevorzugt von jedem von *Enterococcus faecium, Lactobacillus plantarum* und entweder *Lactobacillus casei* oder *Lactobacillus rhamnosus* oder einer Kombination von *Lactobacillus casei* und *Lactobacillus rhamnosus* in der Zubereitung wenigstens 1,0 x 10⁷ lebensfähige Zellen pro Milliliter ist.

6. Probiotische Zubereitung zur Verwendung nach einem der vorhergehenden Ansprüche, wobei die probiotische Zubereitung einen Gesamtkohlenhydratgehalt im Bereich von 20 mg/ml bis 40 mg/ml und einen Gehalt an reduzierendem Zucker im Bereich von 5 mg/ml bis 20 mg/ml aufweist.

7. Probiotische Zubereitung zur Verwendung nach einem der vorhergehenden Ansprüche, die zusätzlich Proteine und Peptide umfasst, wobei der Gesamtgehalt an Protein und Peptiden im Bereich von 1 mg/ml bis 2 mg/ml ist und wobei der Gesamtgehalt an Peptiden mit hohem Molekulargewicht im Bereich von 100 µg/ml bis 300 µg/ml ist.

8. Probiotische Zubereitung zur Verwendung nach einem der vorhergehenden Ansprüche, wobei der pH der Zubereitung im Bereich von 3,8 bis 4,5 gehalten ist.

9. Probiotische Zubereitung zur Verwendung nach einem der vorhergehenden Ansprüche, wobei die probiotische Zubereitung durch Wachsen des probiotischen Bakterienstamms/der probiotischen Bakterienstämme in einem Extrakt von gekeimter Gerste zubereitet ist.

10. Probiotische Zubereitung zur Verwendung nach einem der vorhergehenden Ansprüche, wobei die Zubereitung ferner ein antimykotisches Mittel umfasst, bevorzugt wobei das antimykotische Mittel sterilisiertes Kaliumsorbat ist.

11. Probiotische Zubereitung zur Verwendung nach einem der vorhergehenden Ansprüche, wobei die Zubereitung ferner ein Antioxidans umfasst, bevorzugt wobei das Antioxidans Vitamin C ist.

12. Probiotische Zubereitung zur Verwendung nach einem vorhergehenden Anspruch, zur Verwendung bei der Behandlung von Patienten, bei denen Rom-III klassifiziertes Reizdarmsyndrom diagnostiziert ist.

13. Probiotische Zubereitung zur Verwendung nach einem vorhergehenden Anspruch, zur Verwendung bei der Behandlung von Patienten, bei denen Reizdarmsyndrom diagnostiziert ist, bei denen Erst- und Zweitlinienbehandlungen des Reizdarmsyndroms gescheitert sind.

14. Probiotische Zubereitung zur Verwendung nach einem vorhergehenden Anspruch, zur Behandlung von Patienten mit einem Symptomschweregrad des Reizdarmsyndroms von mehr als 150.

## Revendications

1. Préparation probiotique comprenant des bactéries probiotiques, métaboliquement actives, viables dans un substrat liquide non laitier, destinée à être utilisée dans le traitement du syndrome de l'intestin irritable, où la préparation probiotique comprend un mélange d'*Enterococcus faecium,* de *Lactobacillus plantarum,* de *Lactobacillus acidophilus* et soit de *Lactobacillus casei* soit de *Lactobacillus rhamnosus* ou à la fois *Lactobacillus casei* et *Lactobacillus rhamnosus.*

2. Préparation probiotique destinée à être utilisée selon la revendication 1, la préparation probiotique comprenant un mélange de glucides complexes et de sucres simples, où le rapport de la teneur en glucides totaux sur la teneur en sucres réducteurs de la préparation probiotique se trouve dans la plage allant de 8:1 à 2:1.

3. Préparation probiotique destinée à être utilisée selon l'une quelconque des revendications précédentes, dans laquelle le nombre total de bactéries métaboliquement actives dans la préparation se trouve dans la plage allant de 1,0 x 10⁶ à 1,0 x 10⁹ cellules viables par millilitre.

4. Préparation probiotique destinée à être utilisée selon la revendication 1, dans laquelle le nombre total d'au moins l'une de, et plus préférablement de chacune d'*Enterococcus faecium,* de *Lactobacillus plantarum* et soit de *Lactobacillus casei* soit de *Lactobacillus rhamnosus* ou d'une combinaison de *Lactobacillus casei* et de *Lactobacillus rhamnosus* dans la préparation est d'au moins 1,0 x 10⁶ cellules viables par millilitre.

5. Préparation probiotique destinée à être utilisée selon la revendication 1, dans laquelle le nombre total d'au moins l'une de, et plus préférablement de chacune d'*Enterococcus faecium,* de *Lactobacillus plantarum* et soit de *Lactobacillus casei* soit de *Lactobacillus rhamnosus* ou d'une combinaison de *Lactobacillus casei* et de *Lactobacillus rhamnosus* dans la préparation est d'au moins 1,0 x 10⁷ cellules viables par millilitre.

6. Préparation probiotique destinée à être utilisée selon l'une quelconque des revendications précédentes, la préparation probiotique ayant une teneur en glucides totaux dans la plage allant de 20 mg/ml à 40 mg/ml et une teneur en sucres réducteurs dans la plage allant de 5 mg/ml à 20 mg/ml.

7. Préparation probiotique destinée à être utilisée selon l'une quelconque des revendications précédentes, qui comprend en outre des protéines et des peptides, où la quantité totale de protéine et de peptides se trouve dans la plage allant de 1 mg/ml à 2 mg/ml et où la quantité totale de peptides de poids moléculaire élevé se trouve dans la plage allant de 100 µg/ml à 300 µg/ml.

8. Préparation probiotique destinée à être utilisée selon l'une quelconque des revendications précédentes, dans laquelle le pH de la préparation est maintenu dans la plage allant de 3,8 à 4,5.

9. Préparation probiotique destinée à être utilisée selon l'une des revendications précédentes, la préparation probiotique étant préparée en faisant croître la/les souches bactériennes probiotiques dans un extrait d'orge germé.

10. Préparation probiotique destinée à être utilisée selon l'une quelconque des revendications précédentes, la préparation comprenant en outre un agent antifongique, de préférence où l'agent antifongique est le sorbate de potassium stérilisé.

11. Préparation probiotique destinée à être utilisée selon l'une quelconque des revendications précédentes, la préparation comprenant en outre un antioxydant, de préférence où l'antioxydant est la vitamine C.

12. Préparation probiotique destinée à être utilisée selon l'une des revendications précédentes, destinée à être utilisée dans le traitement de patients diagnostiqués avec le syndrome de l'intestin irritable classé selon les critères Rome III.

13. Préparation probiotique destinée à être utilisée selon l'une des revendications précédentes, destinée à être utilisée dans le traitement de patients diagnostiqués avec le syndrome de l'intestin irritable pour lesquels les traitements de première et deuxième intentions pour le syndrome de l'intestin irritable ont échoué.

14. Préparation probiotique destinée à être utilisée selon l'une des revendications précédentes, pour le traitement des patients présentant un score de gravité des symptômes du syndrome de l'intestin irritable supérieur à 150.
